# EUROPEAN PATENT SPECIFICATION

(11) **EP 2 223 103 B1**
(45) Date of publication and mention of the grant of the patent: **22.01.2020**
(21) Application number: 08858352.1
(22) Date of filing: 28.11.2008
(51) Int. Cl.: A61B 10/00, B01L 3/02, B01L 3/00

(54) **FLUID SAMPLE COLLECTING AND ANALYZING APPARATUS**
FLÜSSIGKEITSPROBENSAMMEL- UND -ANALYSEVORRICHTUNG
APPAREIL DE PRÉLÈVEMENT ET D'ANALYSE D'UN ÉCHANTILLON DE LIQUIDE

(30) Priority: 29.11.2007 US 998610
(43) Date of publication of application: 01.09.2010
(73) Proprietor: Ameditech, Inc., San Diego, CA 92121 (US)
(72) Inventor: WU, John, San Diego CA 92121 (US); NG, Waiping, San Diego CA 92121 (US)
(74) Representative: Hoffmann Eitle
(86) International application number: PCT/US2008/013229
(87) International publication number: WO 2009/073155

(56) References cited:
- EP-A1- 0 074 842
- EP-A1- 1 847 321
- EP-A1- 1 847 321
- WO-A1-93/14798
- WO-A1-94/04078
- US-A1- 2002 106 809
- US-A1- 2007 026 530
- US-B2- 6 565 808

## Description

### FIELD OF THE INVENTION

The present invention relates to devices and methods for collecting and analyzing body fluids for the presence of one or more analytes by immunoassay testing techniques. More specifically, the invention relates to devices for collecting, testing, temporarily storing and transporting saliva samples.

### BACKGROUND

Testing for substance abuse has become standard procedure in a variety of settings such as employment, schools, sports, and law enforcement. The industry has so far provided a great variety of easy-to-use collecting and testing devices which can be utilized by technicians with limited training in the field for preliminary test purposes.

Due to the speed at which the devices of the prior art are used, the relative lack of sophistication of the technicians, and the less than propitious environment of the test, it has become desirable, if not mandatory, to preserve an aliquot of the sample fluid for further testing and validation under more controlled conditions to seek to confirm the results of the earlier preliminary test.

U.S. Patent Application Publication No. US2005/0202568 A1 Tung et al. dated Sep. 15, 2005 discloses a sophisticated device which provides for the preservation of part of the fluid specimen in a sealed plastic reservoir for confirmatory testing at a later date. Such a sophisticated device can be costly to manufacture, requires more operator skill and time to use, and is more susceptible to manufacturing or use errors.

It is well known that small amounts of analytes, such as those associated with marijuana or other abused drugs, can be adsorbed over time on the surfaces of fluid specimen containers. Such adsorption reduces the concentration of analytes remaining in the specimen leading to inaccuracies in testing results associated with detection of those analytes. This problem can be exacerbated by containers used to transport specimens for confirmatory testing where the specimen remains in the container for longer periods and is subjected to agitation and temperature changes during transport. It is therefore desirable to preserve an aliquot of the fluid specimen in such a way as to minimize adsorption of target analytes on the aliquot container surfaces.

EP 1 847 321 A1 shows a sample collector and test device according to the preamble of claim 1. WO 94/04078 A1 is concerned with an oral collection device and kit.

The invention results from an attempt to devise a simpler apparatus that reduces some of the above identified problems.

### Summary

The principal and secondary objects of the invention are to provide a simpler collection and test apparatus that preserves part of a fluid sample for later processing. These and other objects are achieved by an apparatus as defined in claim 1.

In some embodiments there is provided a fluid sample collecting and testing device having a spongy cylindrical swab is axially mounted against the distal face of a piston actioned by a push rod. In some embodiments once humected with a sample fluid such as saliva, the swab-and-piston assembly is inserted into a tubular body like a plunger into a syringe. In some embodiments as the swab is pushed and partially squeezed against the distal end of the tubular body, and up to a trippable barrier, part of the fluid is excreted into a immunoassay device through a radial aperture in the distal section of the body. In some embodiments the remainder of the sample is kept in a sealed chamber between the piston and the closed distal end of the body until it is excreted through a second aperture until then sealed by a breakable barrier directly into another immunoassay testing device.

In some embodiments the apparatus comprises a plunger including a push rod, a piston attached to one extremity of the push rod, said piston having a distal face, and a spongy swab axially mounted on the distal face; a tubular body open at a first end, closed at an opposite second end, and being dimensioned to be intimately engaged by the piston; the body having at least one exit port proximate the second end; and a trippable barrier positioned to stop the piston distally from the second end; and wherein the swab is dimensioned to be partially squeezed between the distal face of the piston and the second end of the body when the distal face reaches its stopping location determined by the trippable barrier.

In some embodiments the apparatus further comprises an immunoassay testing device connected to the exit port. In some embodiments the exit port is radially positioned proximately distant from the stopping location of the piston. In some embodiments the body has a second exit port located distally from the stopping location, and further includes a breakable stopper closing the second exit. In some embodiments the apparatus also comprises a second immunoassay testing device detachably securable to the second exit port. In some embodiments the trippable barrier comprises at least one radial protrusion on the plunger positioned to contact the tubular body when the distal face of the piston reaches the stopping location. In some embodiments the protrusion is manually compressible or removeable. In some embodiments, the exit port is axially positioned in the second end of the body. In some embodiments the exit port is closed by a breakable seal. In some embodiments the trippable barrier is tripped by increased forced insertion of said plunger into the body. In some embodiments the piston may comprise a peripheral O-ring. In some embodiments the body has a circumferential inside groove having an hemispherical cross-section commensurate with the O-ring; whereby the engagement of the O-ring into the groove constitutes the trippable barrier. In some embodiments the exit port is radially located within the groove. In some embodiments a threaded coupling is provided between the exit port and the testing device.

In some embodiments essentially the collecting and testing apparatus comprises: a syringe-like plunger and tubular body assembly, the tubular body having a closed distal end; a spongy swab distally mounted on the plunger; a trippable barrier preventing the swab from being completely squeezed between the plunger and the distal end of the tubular body; and an analyte testing device connected to an exit port in a distal region of the tubular body.

In some embodiments there is provided a method for conducting a first preliminarily test on a fluid sample while preserving a portion of said sample for later testing, and dispensing said portion during said later testing, said method comprises: collecting said fluid sample upon a spongy swab distally mounted on a syringe-like plunger; extracting a first partial portion of said sample from said swab, thereby leaving a second partial portion of said sample on said swab; directing said first partial portion onto a fluid test device capable of detecting said analyte to obtain a preliminary detection; hermetically enclosing said swab in an openable chamber; forming an opening in said chamber; and, excreting said second partial portion through said opening. In some embodiments said extracting comprises: inserting said plunger a first distance into a tubular body assembly having a closed distal end; preventing insertion of said plunger beyond said first distance using a trippable barrier; tripping said trippable barrier; further inserting said plunger beyond said first distance, thereby causing said excreting.

### Brief Description of the Drawings

**Figure 1** is an illustrative cross-sectional view of a first embodiment of the collecting and testing apparatus according to the invention shown in a first fluid extracting mode.
**Figure 2** is a similar view with the apparatus shown in a post first extraction mode.
**Figure** 3 is an illustrative cross-sectional end view of an alternate embodiment having a widened and flattened push-rod.
**Figure 4** is an illustrative cross-sectional view of an alternate embodiment of the testing apparatus having an end-mountable immunoassay device.
**Figure 5** is an illustrative cross-sectional view of an alternate embodiment of the testing apparatus for use as a specimen collection, transport and dispensing syringe.
**Figures 6 and** 7 illustrate a second type of trippable barrier.
**Figure 8** illustrates a third type of trippable barrier.
**Figure 9** illustrates a fourth type of trippable barrier.
**Figure 10 and 11** illustrate a fifth type of trippable barrier.

### Description of the Exemplary Embodiments

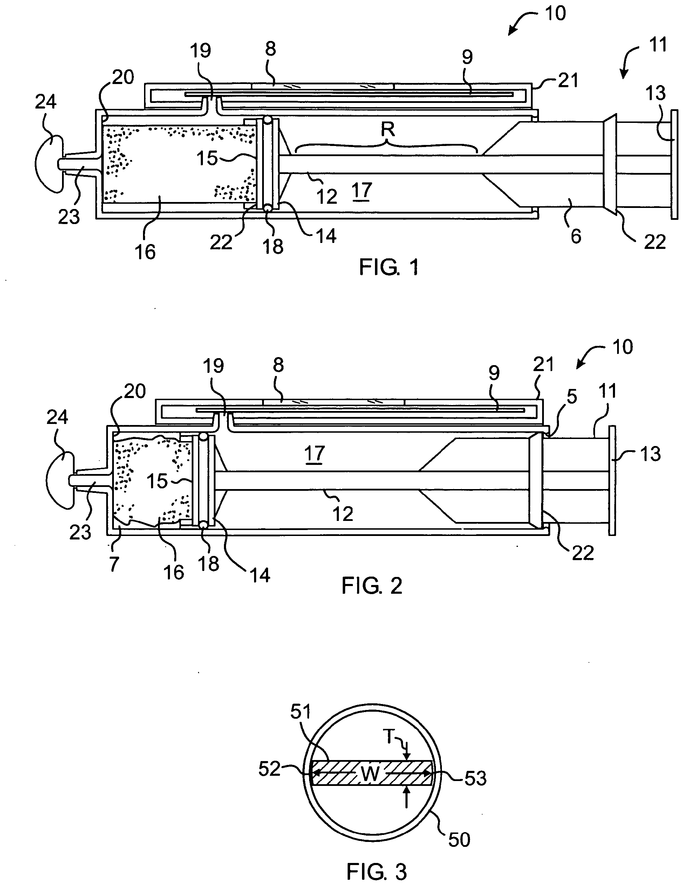

Referring now to the drawing, there is shown in Figures 1 and 2, an apparatus **10** for collecting, testing, storing and transporting a fluid sample such as a saliva specimen. The apparatus comprises a plunger **11** including a push rod **12** having a thumb rest **13** at its proximal end and a piston 14 at the distal end of the rod. The piston has a distal face **15** against which is axially attached a cylindrical spongy swab **16.** The plunger is first used to collect the fluid sample by contact with the swab.

The push rod **12** is shaped and dimensioned to have a medial lip-rest region **R** which allows a user to comfortably close her lips upon it while the swab remains in the user's mouth. The axial length of the lip-rest region is selected to accommodate the closed lips and teeth of a typical adult user. In such circumstances it has been found that the length is preferably between about 2.54 centimeters (1.0 inch) and 5.08 centimeters (2.0 inches), more preferably between about 3.175 centimeters (1.25 inches) and 4.445 centimeters (1.75 inches), and most preferably about 3.81 centimeters (1.5 inches).

The plunger is then inserted and translated axially in a tubular body **17** which is dimensioned to be intimately engaged by the piston **14.** An O-ring **18** peripherally mounted on the wall of the piston provides an hermetical seal between the inside of the tubular body and the piston. A radial exit port **19** is located proximately distant from the end wall **20** of the tubular body, that is ahead of the end wall. A centering element **6** is located distally from the thumb rest **13** to helps maintain a coaxial relationship between the plunger **11** and body **17** during the forced insertion of the plunger. In order to accommodate these forces, the location of the centering element is selected to cause it to engage the tubular body before compression of the swab begins. A second exit port **23** axially practiced in the end wall **20** is sealed by a breakable stopper **24.**

As the plunger is pushed into the tubular body, the spongy swab **16** is squeezed between the distal face of the piston **15** and the end wall **20.** The part of the sample fluid within the swab is excreted through the exit port **19** and into a chromatographic immunoassay test device **21** secured to and in fluid communication with the exit port **19.** The test device **21** is adapted to carry one or more chromatographic testing strips **9** viewable through an exposed window **8** according to techniques well-known in the bio-medical arts.

As shown in Figure 2, when the penetration of the plunger proceeds beyond where the piston O-ring **18** has passed the exit port **19,** the swab containing the aliquot of fluid specimen becomes hermetically enclosed in a chamber **7** formed in the tubular body between the end wall and piston. The small amount of remaining gas in the chamber compresses but the fluid does not, effectively preventing further penetration of plunger beyond a stopping position illustrated in Figure 2, at a point where the swab **16** is not completely squeezed and still contains an aliquot of fluid specimen. The radial exit port **19** is proximally distant, i.e. just ahead from the point where the distal face **15** of the piston is temporarily stopped. This arrangement acts as a barrier to further penetration due to the pressure increase in the chamber when further penetration is attempted.

A compressible barrier **22** in the form of a rubber radial projection such as a retaining ring glued to the plunger passes over the lip **5** at the proximal end of the tubular body and indicates to the operator that penetration has proceeded to an adequate degree. The ring is flared outwardly toward the proximal end so as to discourage extraction of the plunger from the body. In addition the enclosure creates what amounts to a vacuum seal which can help to prevent the extraction of the plunger. In this way preservation of the aliquot is enhanced over other designs in which the reverse motion of the plunger is allowed.

The swab is biased toward its uncompressed state. In this way the compressed swab tends to substantially fill the chamber **7** further discouraging fluid circulation during transport. That aliquot is preserved within the swab which prevents circulation of the aliquot against the container surfaces thereby discouraging loss of analytes due to adsorption on the container surfaces. The swab material also acts to thermally insulate the preserved fluid against temperature changes thereby further decreasing analyte loss through adsorption.

The barrier is trippable, that is, it may be overcome by removal of the stopper **24** and forced further penetration of the plunger into the tubular body through application of additional pressure against the thumb rest **13.** The remaining fluid specimen can thus be extracted at a later date to conduct a confirming test by breaking the stopper **24,** and forcefully pushing the piston as far as is necessary to fully compress the swab **16.** It should be understood that the dimensions of the tubular body and location of the radial exit port are selected so that fluid pressure in the chamber can substantially prevent further compression of the swab until the stopper is removed, and thereby act as a trippable barrier.

Because of the number of functions which are provided by the plunger, care must be taken in determining the relative sizes and shapes of the component structures. For example, the push rod should have low bulk to accommodate comfortable closure of the users lips upon it while still providing enough material to provide a strength to withstand the forces of insertion into the tubular body and forced compression of the swab.

Alternately, the push rod can be formed to have an elongated, flattened shape to enhance its strength while still providing adequate clearance and comfort to the user. As shown in Figure 3, the width **W** of the push rod **50** is flattened and widened to be commensurate with the inner diameter of the tubular body **51.** Further, the lateral edges **52,53** of the rod can be curved to intimately bear against the curved inner surface of the tubular body. The thickness **T** is minimized while still affording adequate resistence to bending and column strength to the push rod. This shape allows for the users lips to be comfortably closed thereon in the medial lip-rest region and act as a centering element, if necessary, along the entire length of the push rod.

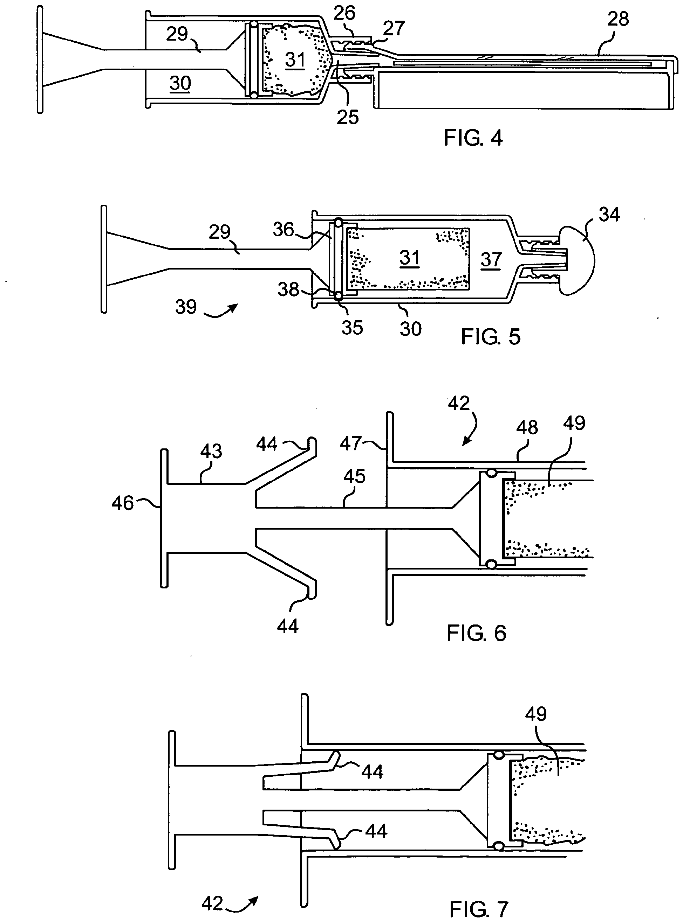

Referring now to Figure 4, there is shown a second embodiment of the invention in which no radial exit port is provided as in the first embodiment. Instead, an axial exit port **25** in the end wall has a threaded coupling 26 that mates with the inlet coupling **27** of a detachable immunoassay device **28.**

As in the first embodiment, the progress of the plunger **29** within the tubular body **30** is first stopped at a given distance from the end wall either by a trippable barrier (as shown in Figs. 6-11) or purposefully by the operator. Accordingly, a first test device may be used to receive and preliminarily analyze a portion of the specimen fluid extracted by the first partial compression of the swab **31,** and a second test device may be used later on to analyze the remainder of the sample fluid excreted by the complete squeezing of the swab **31.** It should be understood that the immunoassay device **28** can be detached at the threaded coupling and replaced with a threaded cap, such as the cap **34** shown in Figure 5, to hermetically enclosed the aliquot and allow its transport to a different location for excretion.

Referring now to Figure 5, it should be noted that the plunger **29** carrying the swab **31** and the tubular body **30** along with a stopper **34** can be provided separately without any immunoassay device to allow for collection, storage and transport of the fluid specimen to a lab where testing occur. In this way, the plunger and swab are used to collect a specimen. The plunger **29** is then inserted into the body **30** having the stopper **34** already in place. The stopper can be of the threaded cap type, as shown, or of the breakable type shown in the embodiment of Figures 1 and 2. The plunger engages the body until the O-ring **38** engages a circumferential groove **35** set into the inside wall of the body. The groove has a hemispherical cross-section commensurate with the cross-section of the O-ring **38** that surrounds the piston **36.** This encloses the specimen carried on the swab in a hermetically sealed chamber **37** formed between the plunger and the body. The engagement of the O-ring into the groove, coupled with the increase in pressure of the chamber acts as a trippable barrier to the further insertion of the plunger. The combined structure **39** of the plunger and body can then be transported to a lab or other location where the stopper can be removed and an immunoassay device of the type shown in Figure 4 attached and testing carried out.

It is important to note that with the stopper removed, the combined structure can be conveniently used by a lab operator as a syringe to dispense the specimen fluid into a number of various devices.

A second type of trippable barrier **42** is illustrated in Figures 6 and 7. The plunger **43** is provided with a series of peripheral prongs **44** which skirt the push rod **45** at a short distance from the thumb rest **46.** The prongs flexibly flare out to form an impediment to the progression of the plunger when they come in contact with the rim **47** of the tubular body **48.** The prongs can be deactivated by being compressed between thumb and index finger so that they will penetrate the tubular body as shown in Figure 6 allowing the plunger to further push against the swab **49.**

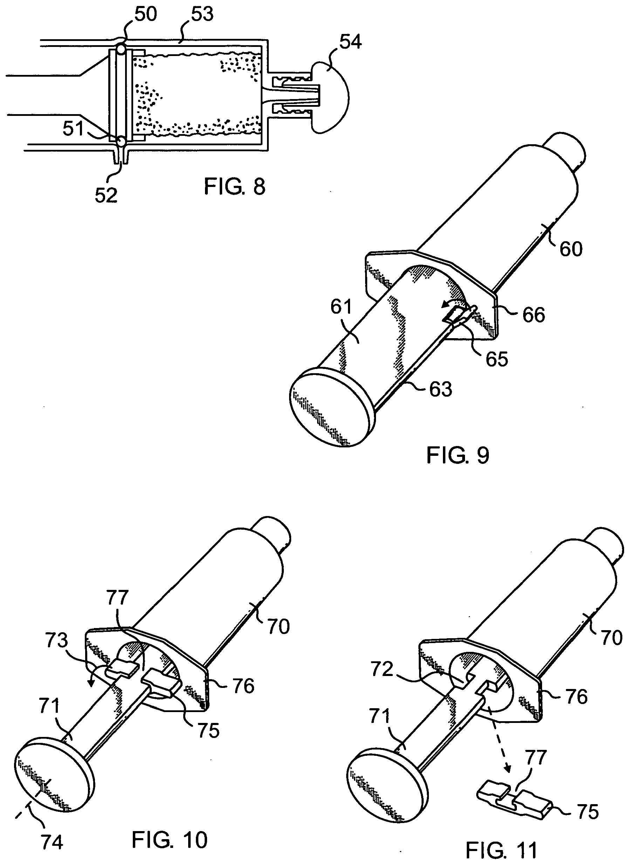

A third type of trippable barrier is illustrated in Figure 8. A circumferential groove **50** is provided in the inside wall of the tubular body **53.** The groove has a hemispherical cross-section commensurate with the cross-section of the O-ring **51** that surrounds the piston. The radial exit port **52** corresponding to the exit port **19** of Figure 1 is located within the groove. When the O-ring, which is normally slightly compressed by the walls of the tubular enclosure, reaches the groove **50** it expands into it. This expansion stops the progress of the piston at the location corresponding to the point necessary to squeeze out a part of the sample fluid. In addition, the O-ring provides a positive closure of the radial exit port **52.** The remainder of the fluid in the swab is now preserved in a sealed chamber between the piston and the end wall of the tubular enclosure. By removing the end stopper **54** and applying additional or increased pressure on the plunger, the barrier can be overcome in order to extract the remainder of the fluid specimen through the exit port at the end of the body.

A fourth type of trippable barrier is shown in Figure 9. A rigid flap **65** is hingedly mounted to the lateral edge **63** of a push-rod **61.** In its open position (shown) the flap acts as a radial prominence which contacts the rim **66** of the tubular body **60** to prevent further penetration. The flap is deactivated by folding it inward against the rod allowing penetration to proceed.

A fifth type of trippable barrier is shown in Figures 10-11. A detachable winglet **75** is mounted within a cradle **72** formed by two indentations set into the opposite lateral edges of a medial section of a push-rod **71** having a flattened, oblong cross-section. In its mounted position (shown in Figure 10) the winglet **75** acts as a radial prominence which contacts the rim **76** of the tubular body **70** to prevent further penetration. The winglet is detached by rotating it angularly **73** with respect to the insertion axis **74** of the plunger so that a notch **77** on the upper medial edge of the winglet aligns with a narrower dimension of the cradle of the flattened push-rod **71.** Alternately, the winglet can be made from a slightly resilient material which allows the notch to be widened to facilitate removal of the winglet from the push-rod.

While the preferred embodiments of the invention have been described, modifications can be made and other embodiments may be devised without departing from the scope of the appended claims.

## Claims

1. A collecting and testing apparatus (10), for detecting an analyte in a fluid sample, wherein the collecting and testing apparatus comprises:
a plunger (11) including a push rod (12), a piston (14) attached to one extremity of said rod, said piston having a distal face (15), and a spongy swab (16) axially mounted on said distal face;
a tubular body open (17) at a first end, closed at an opposite second end (20), and being dimensioned to be intimately engaged by said piston; and
said body having at least one exit port(19, 23) proximate said second end; **characterised by**:
a trippable barrier (22, 24) positioned to stop said piston at a first distance from said second end; and
wherein said swab is dimensioned to be partially squeezed between said distal face and said second end when said distal face reaches a stopping location determined by said trippable barrier.

2. The apparatus of claim 1 which further comprises an immunoassay testing device (21) connected to said exit port.

3. The apparatus of claim 2, wherein said exit port is radially positioned proximally from said stopping location.

4. The apparatus of claim 3, wherein said body has a second exit port (23) located distally from said stopping location; and
further includes a breakable stopper (24) closing said second exit.

5. The apparatus of claim 4 which further comprises a second immunoassay testing device (28) detachably securable to said second exit port.

6. The apparatus of claim 1, wherein said trippable barrier comprises at least one radial protrusion (22, 24, 51, 75) on said plunger, said protrusion being positioned to contact said body when said distal face reaches said stopping location.

7. The apparatus of claim 6, wherein said protrusion (75) is manually removeable.

8. The apparatus of claim 2, wherein said exit port (23, 25) is axially positioned in said second end.

9. The apparatus of claim 8 which further comprises a breakable seal (24, 34) closing said exit port.

10. The apparatus of claim 1, wherein said trippable barrier (52, 51) is trippable by increased forced insertion of said plunger into the body.

11. The apparatus of claim 1, wherein said piston comprises a peripheral O-ring (18, 38, 51).

12. The apparatus of claim 11, wherein said body has a circumferential inside groove (50) having a hemispherical cross-section commensurate with said O-ring; whereby the engagement of said O-ring into said groove constitutes said trippable barrier.

13. The apparatus of claim 12, wherein said exit port (52) is radially located within said groove.

14. The apparatus of claim 8 which further comprises a threaded coupling (26, 27) between said exit port (25) and said testing device (28).

15. The apparatus of Claim 1 which further comprises means for preventing circulation of an amount of said specimen within said body.

16. The apparatus of any preceding claim, said trippable barrier for preventing said swab from being completely squeezed between said plunger and said second end

17. A method for conducting a first preliminarily test on a fluid sample while preserving a portion of said sample for later testing, and dispensing said portion during said later testing, the method using the apparatus of claim 1, said method comprises:
collecting said fluid sample upon a spongy swab (16) distally mounted on a syringe-like plunger (11);
extracting a first partial portion of said sample from said swab, thereby leaving a second partial portion of said sample on said swab;
directing said first partial portion onto a fluid test device (21) capable of detecting said analyte to obtain a preliminary detection;
hermetically enclosing said swab in an openable chamber (7);
forming an opening (23) in said chamber; and,
excreting said second partial portion through said opening, wherein said extracting comprises inserting said plunger a first distance into a tubular body assembly having a closed distal end (20); the method comprising:
preventing insertion of said plunger beyond said first distance using a trippable barrier (22, 24); tripping said trippable barrier;
further inserting said plunger beyond said first distance, thereby causing said excreting.

18. The apparatus of any one of claims 1 to 16 for conducting a first preliminarily test on the fluid sample while preserving a portion of said sample for later testing, and dispensing said portion during said later testing,
the spongy swab for collecting said fluid sample;
the plunger insertable the first distance into the tubular body, wherein the trippable barrier is for preventing insertion of said plunger beyond said first distance so as to extract a first partial portion of said sample from said swab, thereby leaving a second partial portion of said sample on said swab and so as to direct said first partial portion onto a fluid test device (21) capable of detecting said analyte to obtain a preliminary detection;
wherein at the stopping location, a sealed, openable chamber, is defined between the distal face of the piston and the second end of the body for hermetically enclosing said swab in the openable chamber (7);
an opening (23) formable in said chamber, and
wherein the trippable barrier is trippable to allow further insertion of said plunger beyond said first distance, thereby causing excreting of said second partial portion through said opening.

## Patentansprüche

1. Sammel- und Testeinrichtung (10) zum Nachweisen eines Analyten in einer Flüssigkeitsprobe, wobei die Sammel- und Testeinrichtung umfasst:
einen Plunger (11), der eine Schubstange (12) einschließt, einen Kolben (14), der an einem Ende der Stange befestigt ist, wobei der Kolben eine distale Fläche (15) aufweist, und einen schwammartigen Tupfer (16), der axial auf der distalen Fläche angebracht ist;
einen rohrförmigen Körper, der an einem ersten Ende offen (17) ist, an einem gegenüberliegenden zweiten Ende (20) geschlossen ist und so bemessen ist, dass er mit dem Kolben eng in Eingriff steht; und
wobei der Körper mindestens eine Austrittsöffnung (19, 23) nahe dem zweiten Ende aufweist; **gekennzeichnet durch**:
eine auslösbare Barriere (22, 24), die so positioniert ist, dass sie den Kolben in einem ersten Abstand von dem zweiten Ende stoppt; und
wobei der Tupfer so bemessen ist, dass er teilweise zwischen der distalen Fläche und dem zweiten Ende zusammengedrückt wird, wenn die distale Fläche eine durch die auslösbare Barriere bestimmte Stoppstelle erreicht.

2. Einrichtung nach Anspruch 1, die weiter eine Immunoassay-Testvorrichtung (21) umfasst, die mit der Austrittsöffnung verbunden ist.

3. Einrichtung nach Anspruch 2, wobei die Austrittsöffnung radial proximal von der Stoppstelle positioniert ist.

4. Einrichtung nach Anspruch 3, wobei der Körper eine zweite Austrittsöffnung (23) aufweist, die sich distal von der Stoppstelle befindet; und
weiter einen brechbaren Stopfen (24) einschließt, der den zweiten Ausgang verschließt.

5. Einrichtung nach Anspruch 4, die weiter eine zweite Immunoassay-Testvorrichtung (28) umfasst, die lösbar an der zweiten Austrittsöffnung befestigbar ist.

6. Einrichtung nach Anspruch 1, wobei die auslösbare Barriere mindestens einen radialen Vorsprung (22, 24, 51, 75) auf dem Plunger umfasst, wobei der Vorsprung so positioniert ist, dass er den Körper berührt, wenn die distale Fläche die Stoppstelle erreicht.

7. Einrichtung nach Anspruch 6, wobei der Vorsprung (75) manuell entfernbar ist.

8. Einrichtung nach Anspruch 2, wobei die Austrittsöffnung (23, 25) axial in dem zweiten Ende positioniert ist.

9. Einrichtung nach Anspruch 8, die weiter eine brechbare Dichtung (24, 34) umfasst, welche die Austrittsöffnung verschließt.

10. Einrichtung nach Anspruch 1, wobei die auslösbare Barriere (52, 51) durch verstärktes forciertes Einführen des Plungers in den Körper auslösbar ist.

11. Einrichtung nach Anspruch 1, wobei der Kolben einen periphären O-Ring (18, 38, 51) umfasst.

12. Einrichtung nach Anspruch 11, wobei der Körper eine umlaufende Innennut (50) aufweist, die einen halbkugelförmigen Querschnitt aufweist, der dem O-Ring entspricht; wobei das Eingreifen des O-Rings in die Nut die auslösbare Barriere bildet.

13. Einrichtung nach Anspruch 12, wobei sich die Austrittsöffnung (52) radial innerhalb der Nut befindet.

14. Einrichtung nach Anspruch 8, die weiter eine Gewindekupplung (26, 27) zwischen der Austrittsöffnung (25) und der Testvorrichtung (28) umfasst.

15. Einrichtung nach Anspruch 1, die weiter Mittel zum Verhindern der Zirkulation einer Menge der Probe innerhalb des Körpers umfasst.

16. Einrichtung nach einem der vorstehenden Ansprüche, wobei die auslösbare Barriere verhindert, dass der Tupfer vollständig zwischen dem Plunger und dem zweiten Ende zusammengedrückt wird.

17. Verfahren zur Durchführung eines ersten vorläufigen Tests an einer Flüssigkeitsprobe unter Erhaltung eines Abschnitts der Probe für einen späteren Test und Abgabe des Abschnitts während des späteren Tests, wobei das Verfahren die Einrichtung nach Anspruch 1 verwendet, wobei das Verfahren umfasst:
Sammeln der Flüssigkeitsprobe auf einem schwammartigen Tupfer (16), der distal auf einem spritzenartigen Plunger (11) angebracht ist;
Extrahieren eines ersten Teilabschnitts der Probe aus dem Tupfer, wodurch ein zweiter Teilabschnitt der Probe auf dem Tupfer verbleibt;
Leiten des ersten Teilabschnitts auf eine Flüssigkeitstestvorrichtung (21), die in der Lage ist, den Analyten nachzuweisen, um einen vorläufigen Nachweis zu erhalten;
hermetisches Einschließen des Tupfers in einer zu öffnenden Kammer (7);
Bilden eines Lochs (23) in der Kammer; und,
Ausscheiden des zweiten Teilabschnitts durch das Loch, wobei das Extrahieren das Einführen des Plungers in einem ersten Abstand in eine Rohrkörperanordnung umfasst, die ein geschlossenes distales Ende (20) aufweist; wobei das Verfahren umfasst:
Verhindern des Einführens des Plungers über den ersten Abstand hinaus unter Verwendung einer auslösbaren Barriere (22, 24);
Auslösen der auslösbaren Barriere;
weiter Einführen des Plungers über den ersten Abstand hinaus, wodurch die Ausscheidung verursacht wird.

18. Einrichtung nach einem der Ansprüche 1 bis 16 zur Durchführung eines ersten vorläufigen Tests an der Flüssigkeitsprobe unter Erhaltung eines Abschnitts der Probe für ein späteres Testen und Abgabe des Abschnitts während des späteren Testens,
wobei der schwammartige Tupfer zum Sammeln der Fluidprobe ist;
wobei der Plunger dem ersten Abstand in den rohrförmigen Körper einführbar ist, wobei die auslösbare Barriere zum Verhindern des Einführens des Plungers über den ersten Abstand hinaus ist, um einen ersten Teilabschnitt der Probe aus dem Tupfer zu extrahieren, wodurch ein zweiter Teilabschnitt der Probe auf dem Tupfer verbleibt, und um den ersten Teilabschnitt auf eine Flüssigkeitstestvorrichtung (21) zu leiten, die in der Lage ist, den Analyten nachzuweisen, um einen vorläufigen Nachweis zu erhalten;
wobei an der Stoppstelle eine abgedichtete, zu öffnende Kammer zwischen der distalen Fläche des Kolbens und dem zweiten Ende des Körpers definiert ist, um den Tupfer hermetisch in der zu öffnenden Kammer (7) zu umschließen;
wobei ein Loch (23) in der Kammer formbar ist, und
wobei die auslösbare Barriere auslösbar ist, um ein weiteres Einführen des Plungers über den ersten Abstand hinaus zu ermöglichen, wodurch die Ausscheidung des zweiten Teilabschnitts durch das Loch verursacht wird.

## Revendications

1. Appareil de collecte et de test (10), pour détecter un analyte dans un échantillon de fluide, dans lequel l'appareil de collecte et de test comprend :
un plongeur (11) incluant une tige de poussée (12), un piston (14) fixé à une extrémité de ladite tige, ledit piston présentant une face distale (15), et un tampon spongieux (16) monté axialement sur ladite face distale ;
un corps tubulaire ouvert (17) à une première extrémité, fermé à une seconde extrémité opposée (20), et dimensionné pour que ledit piston s'y loge intimement ; et
ledit corps présentant au moins un orifice de sortie (19, 23) à proximité de ladite seconde extrémité ; **caractérisé par** :
une barrière déclenchable (22, 24) positionnée pour arrêter ledit piston à une première distance par rapport à ladite seconde extrémité ; et
dans lequel ledit tampon est dimensionné pour être partiellement pressé entre ladite face distale et ladite seconde extrémité quand ladite face distale atteint un emplacement d'arrêt déterminé par ladite barrière déclenchable.

2. Appareil selon la revendication 1 qui comprend en outre un dispositif de test d'immuno-essai (21) connecté audit orifice de sortie.

3. Appareil selon la revendication 2, dans lequel ledit orifice de sortie est positionné radialement de manière proximale par rapport audit emplacement d'arrêt.

4. Appareil selon la revendication 3, dans lequel ledit corps présente un second orifice de sortie (23) situé de manière distale par rapport audit emplacement d'arrêt ; et
inclut en outre un bouchon rupturable (24) fermant ladite seconde sortie.

5. Appareil selon la revendication 4 qui comprend en outre un second dispositif de test d'immuno-essai (28) pouvant être fixé de manière amovible audit second orifice de sortie.

6. Appareil selon la revendication 1, dans lequel ladite barrière déclenchable comprend au moins une saillie radiale (22, 24, 51, 75) sur ledit plongeur, ladite saillie étant positionnée pour être en contact avec ledit corps quand ladite face distale atteint ledit emplacement d'arrêt.

7. Appareil selon la revendication 6, dans lequel ladite saillie (75) est amovible manuellement.

8. Appareil selon la revendication 2, dans lequel ledit orifice de sortie (23, 25) est positionné axialement dans ladite seconde extrémité.

9. Appareil selon la revendication 8 qui comprend en outre un joint d'étanchéité rupturable (24, 34) fermant ledit orifice de sortie.

10. Appareil selon la revendication 1, dans lequel ladite barrière déclenchable (52, 51) est déclenchable par l'insertion forcée croissante dudit plongeur dans le corps.

11. Appareil selon la revendication 1, dans lequel ledit piston comprend un joint torique périphérique (18, 38, 51).

12. Appareil selon la revendication 11, dans lequel ledit corps présente une rainure interne circonférentielle (50) présentant une section transversale hémisphérique en rapport avec ledit joint torique ; selon laquelle l'insertion dudit joint torique dans ladite rainure constitue ladite barrière déclenchable.

13. Appareil selon la revendication 12, dans lequel ledit orifice de sortie (52) est situé radialement à l'intérieur de ladite rainure.

14. Appareil selon la revendication 8 qui comprend en outre un couplage fileté (26, 27) entre ledit orifice de sortie (25) et ledit dispositif de test (28).

15. Appareil selon la revendication 1 qui comprend en outre un moyen pour empêcher la circulation d'une quantité dudit échantillon dans ledit corps.

16. Appareil selon l'une quelconque des revendications précédentes, ladite barrière déclenchable pour empêcher que ledit tampon ne soit complètement pressé entre ledit plongeur et ladite seconde extrémité.

17. Procédé pour effectuer un premier test préliminaire sur un échantillon de fluide tout en conservant une partie dudit échantillon pour un test ultérieur, et pour distribuer ladite partie au cours dudit test ultérieur, ledit procédé utilisant l'appareil selon la revendication 1, ledit procédé comprend :
la collecte dudit échantillon de fluide sur un tampon spongieux (16) monté de manière distale sur un plongeur de type seringue (11) ;
l'extraction d'une première partie partielle dudit échantillon à partir dudit tampon, pour ainsi laisser une seconde partie partielle dudit échantillon sur ledit tampon ;
le fait de diriger ladite première partie partielle sur un dispositif de test de fluide (21) capable de détecter ledit analyte pour obtenir une détection préliminaire ;
l'enfermement de manière hermétique dudit tampon dans une chambre pouvant être ouverte (7) ;
la formation d'une ouverture (23) dans ladite chambre ; et,
l'excrétion de ladite seconde partie partielle à travers ladite ouverture, dans lequel ladite extraction comprend l'insertion dudit plongeur sur une première distance dans un ensemble corps tubulaire présentant une extrémité distale fermée (20); le procédé comprenant :
le fait d'empêcher l'insertion dudit plongeur au-delà de ladite première distance en utilisant une barrière déclenchable (22, 24) ;
le déclenchement de ladite barrière déclenchable ;
le fait d'insérer plus loin ledit plongeur au-delà de ladite première distance, pour ainsi provoquer ladite excrétion.

18. Appareil selon l'une quelconque des revendications 1 à 16 pour effectuer un premier test préliminaire sur l'échantillon de fluide tout en conservant une partie dudit échantillon pour un test ultérieur, et pour distribuer ladite partie au cours dudit test ultérieur,
le tampon spongieux pour collecter ledit échantillon de fluide ;
le plongeur pouvant être inséré la première distance dans le corps tubulaire, dans lequel la barrière déclenchable est pour empêcher l'insertion dudit plongeur au-delà de ladite première distance afin d'extraire une première partie partielle dudit échantillon à partir dudit tampon, pour ainsi laisser une seconde partie partielle dudit échantillon sur ledit tampon et afin de diriger ladite première partie partielle sur un dispositif de test de fluide (21) capable de détecter ledit analyte pour obtenir une détection préliminaire ;
dans lequel au niveau de l'emplacement d'arrêt, une chambre hermétique pouvant être ouverte est définie entre la face distale du piston et la seconde extrémité du corps pour enfermer hermétiquement ledit tampon dans la chambre pouvant être ouverte (7) ;
une ouverture (23) pouvant être formée dans ladite chambre, et
dans lequel la barrière déclenchable peut être déclenchée pour permettre d'insérer plus loin ledit plongeur au-delà de ladite première distance, pour ainsi provoquer l'excrétion de ladite seconde partie partielle à travers ladite ouverture.
